# EUROPÄISCHE PATENTSCHRIFT

(11) **EP 0 940 663 B1**
(45) Veröffentlichungstag und Bekanntmachung des Hinweises auf die Patenterteilung: **06.05.2004**
(21) Anmeldenummer: 99810102.6
(22) Anmeldetag: 08.02.1999
(51) Int. Cl.: G01K 13/00, A61B 3/10, A61B 5/00

(54) **Zeitlicher Temperaturänderungsvorgang am menschlichen Auge**
Temperature variation process at the human eye
Processus de la variation de la température de l'oeil humain

(30) Priorität: 09.02.1998 CH 31898
(43) Veröffentlichungstag der Anmeldung: 08.09.1999
(73) Patentinhaber: HAAG-STREIT AG, CH-3098 Köniz (CH)
(72) Erfinder: Orgül, Selim, 4104 Oberwil (CH); Flammer, Josef, 4102 Binningen (CH)
(74) Vertreter: Roshardt, Werner Alfred, Dipl.-Phys.

(56) Entgegenhaltungen:
- EP-A- 0 048 402
- US-A- 4 312 364
- US-A- 4 402 311
- US-A- 5 800 481
- R. MAPSTONE: "Determinants of corneal temperature" BRIT. J. OPHTHAL., Bd. 52, 1968, Seiten 729-741, XP002124379
- PATENT ABSTRACTS OF JAPAN vol. 017, no. 584 (M-1501), 25. Oktober 1993 & JP 05 172095 A (SANYO ELECTRIC CO LTD), 9. Juli 1993
- PATENT ABSTRACTS OF JAPAN vol. 010, no. 225 (P-484), 6. August 1986 & JP 61 061024 A (SUMITOMO ELECTRIC IND LTD), 28. März 1986

## Beschreibung

Die Erfindung betrifft ein Verfahren zur Bestimmung eines zeitlichen Temperaturänderungsvorgangs gemäss Patentanspruch 1 sowie eine Vorrichtung gemäss dem Oberbegriff des Patentanspruchs 5.

### Stand der Technik

Aus der Veröffentlichung von R. Mapstone, "Determinants of Corneal Temperature", Brit. J. Ophthal. (1968) 52, Seiten 729-741 ist eine Messmethode bekannt, bei der berührungslos der zeitliche Temperaturverlauf auf der Augenhornhaut gemessen wird. Es wird ein Erwärmungsvorgang nach dem Aufbringen eines eisgekühlten Salzwasser-Tropfenstrom (160 bis 180 Tropfen pro Minute) auf die Hornhaut gemessen. Das Aufbringen des gekühlten Salzwassertropfens ist für den Patienten äusserst unangenehm; auch liess die Reproduzierbarkeit der Messresultate zu wünschen übrig.

Aus der US-A 4 402,311 ist ein Endoskop mit einer Infrarot-Strahlungsquelle zur Bestrahlung von Körperhohlräumen (Uterus) bekannt, welche von Krebs befallen sind. Die Erwärmung des Gewebes an den Wänden des betreffenden Körperhohlraumes wird auf eine vorgegebene Temperatur gebracht und mit einem kontaktfreien pyroelektrischen Temperatursensor kontrolliert. Gemäss US-A 4 402,311 ist bei einem pyrolelektrischen Messverfahren eine gechopperte Erwärmungsstrahlung notwendig. Das Choppern wird mechanisch mit einem durch einen Motor in Rotation versetzbaren Flügelrad vorgenommen.

In der EP-A 0 048 402 ist ein Endoskop beschrieben, mit dem Körperhohlräume mittels Mikrowellen auf eine vorgegebene Temperatur zur Zerstörung von Krebszellen erwärmt werden.

In der US-A 4,312,364 ist eine Vorrichtung zur Erwärmung von lebenden Körperbereichen mit elektromagnetischer Strahlung beschrieben. Die Frequenz der elektromagnetischen Strahlung liegt zwischen 0,3 und 2 GHz. Die Strahlung wird über ein als Doppelleitungssystem ausgebildetes Koaxialkabel auf dessen Innenleiter an die betreffende Körperstelle gebracht. Das Koaxialkabel hat ein weiteres Leitersystem, welches um den Innenleiter herum angeordnet ist. Mit diesem weiteren Leitersystem wird die Erwärmung des bestrahlten Gewebes gemessen.

### Darstellung der Erfindung

### Aufgabe der Erfindung

Aufgabe der Erfindung ist es, ein Verfahren aufzuzeigen sowie eine Vorrichtung zu schaffen, welches bzw. welche eine reproduzierbare berührungslose Messung eines zeitlichen Temperaturverlaufes, ausgehend von einer wohldefinierten vorgegebenen Temperatur, gestattet.

### Lösung der Aufgabe

Die oben genannte Aufgabe wird dadurch gelöst, dass eine eine Messfläche beinhaltende Beeinflussungsfläche berührungslos unter Verwendung eines temperierbaren Gasstrahls beblasen wird, und dann die Temperatur bzw. der zeitliche Temperaturverlauf einer innerhalb der Beeinflussungsfläche liegenden Messfläche ebenfalls berührungslos mit einer strahlenphysikalischen Temperaturmesseinheit gemessen wird.

Die Temperaturmessung kann nun nach Erreichen eines Temperaturgleichgewichts und Ausschalten des temperierenden Gasstrahls oder auch bei dessen Einwirkung gemessen werden. Gegenüber einem Aufbringen einer kühlenden Flüssigkeit auf dem Messort hat der Gasstrahl den Vorteil einer bedeutend besseren Temperatureinstellbarkeit. Auch kann die Grösse der zu beblasenden Beeinflussungs- sowie auch der Messfläche einfach vorgegeben werden. Die einmal vorgegebenen Flächenabmessungen verändern sich im Gegensatz zu einem Flüssigkeitstropfen nicht mehr örtlich; sie sind ortsstabil. Es kann aber auch, falls gewünscht, ein Abscannen oder eine vorgegebene zeitliche und örtliche Veränderung der Beeinflussungs- sowie auch der Messfläche vorgenommen werden.

Wird dem Gasstrahl und/oder in der Achse des optischen Abbildungssystems für die berührungslose Temperaturmessung ein nicht oder nur unwesentlicher erwärmender Beleuchtungsstrahl beigegeben, so sind die Flächen auf einfache Art und Weise zu kennzeichnen und damit einfach auszuwählen bzw. einzustellen.

Bevorzugt wird man, sofern die Messungen am Menschen vorgenommen werden, eine Ruhigstellung der betreffenden Flächen vornehmen. Soll die Messung am Auge vorgenommen werden, so wird die "Beblaseinrichtung" sowie die Optik der Messeinrichtung in eine sog. Kopfstütze, welche den menschlichen Kopf fixiert, integriert. Die Lage des Auges wird dann bevorzugt mit einem Fixierlicht fixiert.

Bevorzugt hat die Vorrichtung einen Abstandshalter, der neben der zu beeinflussenden (beblasenden) bzw. auszumessenden Oberfläche angestellt wird. Das optische Abbildungssystem der berührungslos arbeitenden Temperaturmesseinheit sowie der mit der Blaseinheit erzeugte Gasstrahl sind derart gerichtet und ausgebildet, dass die Messfläche innerhalb der durch den Gasstrahl temperierten Beeinflussungsfläche zu liegen kommt.

Wird die Vorrichtung jedoch bei berührungssensitiven Oberflächen, wie z.B. der Augenhornhaut, verbranntem, entzündetem Hautgewebe, ... eingesetzt, so arbeitet man bevorzugt nicht mit einem mechanisch berührenden Element. In diesem Fall kann man eine optische Betrachtungseinheit verwenden, bei der ein exakter Abstand gegeben ist, sofern der betreffende Oberflächenbereich scharf erkennbar ist. Anstelle einer optischen Betrachtung können Lichtstrahlen eingekoppelt werden. Eine exakte Einstellung ist dann gegeben, wenn beispielsweise zwei Strahlen - einer in der optischen Achse der Messoptik und einer in der Gasstrahlachse - sich in einem Punkt auf der Messfläche schneiden. Es können jedoch auch optisch geometrische Figuren verwendet werden, wobei hierbei auch eine Kennzeichnung des Messfeldes sowie des Beeinflussungsfeldes vorgenommen werden können.

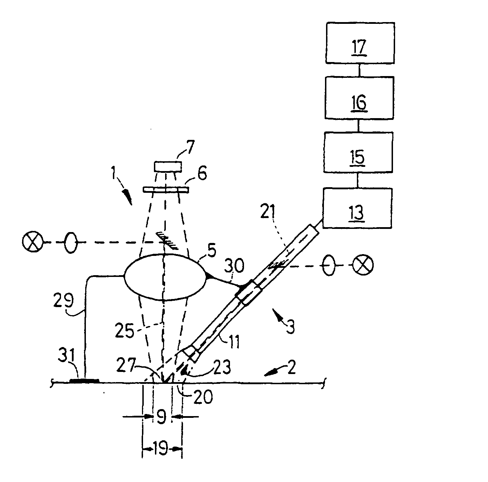

### Kurze Beschreibung der Zeichnung

Nachfolgend werden Beispiele des erfindungsgemässen Verfahrens und der erfindungsgemässen Vorrichtung anhand einer Zeichnung näher erläutert. Die einzige Figur zeigt eine prinzipielle Darstellung der erfindungsgemässen Vorrichtung.

### Wege zur Ausführung der Erfindung

Die in der einzigen Figur dargestellte schematische Darstellung der erfindungsgemässen Vorrichtung zeigt eine berührungslos messende strahlenphysikalische insbesondere hochauflösende Temperaturmesseinheit **1** zur Messung eines zeitlichen Temperaturänderungsvorgangs auf einer Oberfläche **2,** hier beispielsweise der Augenoberfläche, sowie eine Blaseinheit **3.** Die Temperaturmesseinheit **1** hat ein optisches Abbildungssystem **5**, hier schematisch lediglich als Linse dargestellt, eine optische Filtereinheit **6** und ein Detektionselement **7**. Die Messung der Temperatur beruht auf dem spektralen Intensitätsverlauf der mit dem Abbildungssystem **5** auf dem Detektionselement **7** abgebildeten Messfläche **9**. Es wird die von der Messfläche **9** abgestrahlte Wärmestrahlung ausgewertet.

Die Blaseinheit **3** hat ein Blasrohr **11**, das mit einer Temperiereinheit **13** verbunden ist. Die Temperiereinheit **13** ist mit einem mechanischen Filter **15** verbunden, welches seinerseits über eine Gasflussdosiereinheit **16** an ein Gasreservoir **17** angeschlossen ist. Aus dem Gasreservoir **17** wird mit der Dosiereinheit **16** ein vorgegebener Gasfluss eingestellt. Dieser Gasfluss wird mit dem mechanischen Filter **15** von Partikeln befreit und mit der Temperiereinheit **13** auf eine vorgegebene Temperatur gebracht. Anstatt den Gasfluss auf eine vorgegebene konstante Temperatur zu bringen, kann auch ein zeitlicher Temperaturverlauf gewählt werden. Von der Temperiereinheit **13** wird der Gasfluss mit dem Blasrohr **11** zu einem Gasstrahl **20** geformt, der zu einer vorgegebenen Beeinflussungsfläche **19** geführt wird. Durch die Lage der Achse **21** des Blasrohres und der Gestaltung des Rohrausgangs **23** wird die Lage der Beeinflussungsfläche **19** und deren Umfang definiert. Das Gas und die Gasströmung sind derart ausgewählt, dass keine chemischen Reaktionen stattfinden.

Für eine Messung sind die Achse **21** und die optische Achse **25** derart angeordnet, dass ihr Schnittpunkt **27** in der Mitte der Mess- und der Beeinflussungsfläche **9** und **19** liegt. Die Ausgestaltung des optischen Abbildungssystems **5** definiert den Umriss der Messfläche **9**. Die hier definierte Lage des Schnittpunkts **27** in der Mitte der beiden Flächen **9** und **19** wird mechanisch durch einen Abstandshalter **29** vorgegeben. Der Abstandshalter **29** hat ferner eine Verbindungseinheit **30**, mit der das Blasrohr **11** mechanisch fest mit der Temperaturmesseinheit **1** verbunden ist.

Der hier lediglich schematisch dargestellte Abstandshalter **29** stellt ein einfaches und robustes, sicher arbeitendes Bauteil dar, dessen Auflageelement **31** zur Einstellung des für die Durchführung der Messung richtigen Abstands auf der zur Beeinflussungsfläche **19** benachbarten Oberfläche aufgestellt wird. Das Auflageelement **31** kann jedoch bei empfindlichen Mess- und Beeinflussungsflächen **9** und **19**, wie beispielsweise der Augenhornhaut, entzündeten Hautoberflächen etc. nicht verwendet werden. In diesem Fall wird der mechanische Abstandshalter **29** durch eine optische, einen Abstand definierende Einheit ersetzt. Hierzu wird in das optische Abbildungssystem **5** sowie in die Blasrohrachse **21** ein die Mess- und Beeinflussungsflächen **9** und **19** nicht erwärmender Lichtstrahl eingekoppelt. Eine richtige Zuordnung dieser beiden Flächen **9** und **19** zueinander ist dann im Schnittpunkt **27** der beiden Strahlen gegeben.

Zur Überprüfung bzw. zur richtigen Abstandseinstellung des Detektionselements **7** von der Messfläche **9** können nun im Messstrahlengang ebenfalls zwei Strahlen eingeblendet werden, die sich auf der optischen Achse **25** in der Abbildungsebene des Abbildungssystems **5**, in der auch die Messfläche **9** liegt, schneiden. Es kann aber ein einziger Strahl verwendet werden, der mit einem Öffnungswinkel auf die Messfläche **9** fokussiert wird. Der richtige Abstand ist immer dann gegeben, wenn der "Fokusfleck" auf der Messfläche **9** minimal ist. Anstelle eines im Durchmesser minimal einzustellenden Kreises können selbstverständlich auch andere Figuren verwendet werden. Auch können Figuren verwendet werden, deren Konturen nur bei richtigem Abstand scharf sind.

Anstelle eines einzigen Einstellungspunktes **27** können jedoch auch geometrische Figuren verwendet werden. Auch kann über eine optisch scharfe Einstellung einer in das Abbildungssystem eingekoppelten Betrachtungsoptik der richtige Abstand erreicht werden.

Der mit der Temperaturmesseinheit **1** gemessene zeitliche Temperaturverlauf kann mit einer zweiten Temperaturmessung verglichen werden. Hierzu wird man beide Messwerte mit einem Aufzeichnungsgerät aufnehmen und dann auswerten.

Je nach Einsatzzweck der erfindungsgemässen Vorrichtung wird nun der Gasstrahl **20** mit der Temperiereinheit **13** erwärmt oder auch abgekühlt. Als Gas wird man inerte Gase verwenden. Sollen Messung an der menschlichen Haut, der Hornhaut des Auges usw. durchgeführt werden, wird man beispielsweise mit (gekühlter oder erwärmter) Luft, CO₂, Stickstoff oder auch Edelgasen arbeiten. Wird Luft verwendet, wird man sie bevorzugt mit einem Filter reinigen. Als Gasreservoir **17** wird man käufliche Gasflaschen verwenden.

Die Beeinflussungsfläche **19** wird man in der Regel immer grösser als die Messfläche **9** wählen. Es soll hierdurch eine Temperaturhomogenität auf der gesamten Messfläche **9** erreicht werden.

Aus der Reaktion eines Gewebes auf eine Störung, hervorgerufen durch eine Erwärmung oder Abkühlung durch den Gasstrahl **20** sowie aus dem Verhalten nach der Abschaltung des die Störung verursachenden Gasstrahls **20** lassen sich Rückschlüsse auf eventuelle Pathologien ziehen.

Dies gilt insbesondere für okuläres Gewebe. Kühlt man z.B. die Hornhaut mittels einer thermischen Provokation ab, so erreicht sie nach einigen Sekunden eine neue Gleichgewichtstemperatur, welche einige Grad unter der Ausgangstemperatur liegt. Nach Abschalten der Kühlung geht die Hornhauttemperatur aufgrund verschiedener Wärmetransferprozesse (Wärmeleitung über das Hornhautgewebe, Konvektion des Kammerwassers, Wärmetransport durch Blut oder Strahlung, ...) in den Ausgangszustand zurück, sofern sich ansonsten nichts verändert hat. Aus dem zeitlichen Verlauf der Hornhauttemperatur kann auf Pathologien des Gewebes selbst oder der Wärmetransferprozesse geschlossen werden. "Pathologien" bei Wärmetransferprozessen wiederum erlauben Rückschlüsse auf andere okulare Pathologien, wie Durchblutungsstörungen in chorioretinalen Gefässen. Die Reaktion des Augengewebes auf eine Abkühlung mit einem entsprechend temperierten Gasstrahl und die daraus resultierende Reaktion des Augengewebes lassen sich durch Veränderungen der Ausgangsbedingungen, wie z.B. eine Verwendung vasoaktiver (dilatierender/constriktierender) Augentropfen, Augentropfen mit unterschiedlichem Dampfdruck, ... modifizieren. Durch eine Modifikation der Ausgangsbedingungen ist eine Optimierung des Verfahrens möglich. Ebenfalls ist eine Differenzierung pathologischer Wärmetransferprozesse möglich.

Eine Abkühlung der Beeinflussungsfläche **19** wird bevorzugt durch einen entsprechend temperierten Gasstrahl vorgenommen. Eine Erwärmung kann nun ebenfalls mit einem warmen Gasstrahl vorgenommen werden. Zur Erwärmung kann jedoch (nicht erfindungsgemäß) auch eine Wärmestrahlung verwendet werden, die auf die Beeinflussungsfläche **19** mit einem entsprechend ausgelegten Abbildungssystem gerichtet wird.

Zu Korrelationszwecken kann eine weitere Temperaturmessung an einer zweiten Messfläche vorgenommen werden. Die zweite Messfläche ist von der ersten distanziert. Die weitere Temperaturmessung kann ebenfalls berührungslos erfolgen, muss es aber nicht. Werden z.B. die oben beschriebenen Temperaturverlaufsmessungen am menschlichen Auge vorgenommen, so kann die zweite Temperaturmessung an einer der Fingerspitzen vorgenommen werden.

Bei einer Temperaturmessung am Auge wird man die oben beschriebene Messvorrichtung mit einer Kopfhalterung zur Referenzierung des auszumessenden Oberflächenbereichs in Verbindung bringen, d.h. mit einem Trägerteil an dieser befestigen. Eine Fixierung des Auges wird mit einem Fixierlicht vorgenommen. Die Messvorrichtung kann auch an einem ophthalmologischen Spaltlampenmikroskop angeordnet werden. In diesem Fall wird die Temperaturmesseinheit **1** und ein Zielstrahl derart in das Spaltlampenmikroskop integriert, dass der richtige Abstand zum Patientenauge mit dem Zielstrahl eingestellt werden kann. Fixierung und Ausrichtung des Auges erfolgen auch hier über eine Kopfstütze in Kombination mit einem Fixierlicht. In diese Anordnung kann zusätzlich ein Tonometer zur gleichzeitigen Messung des Augendrucks integriert werden. Die Messvorrichtung kann ferner mit komplementären Messgeräten, wie einem Blut-Fluss-Messgerät. einem Fundus-Betrachtungsgerät etc. kombiniert werden, um eine Signifikanz einer Diagnose zu erhöhen.

Anstatt wie vorgehend beschrieben lediglich die Temperatur der gesamten Messfläche **9** zu ermitteln, kann auch die örtliche Temperaturverteilung auf der Messfläche **9** ermittelt werden. Zur Ermittlung der örtlichen Temperaturverteilung kann man entweder ein infrarotempfindliches Aufnahmeelement (CCD) verwenden oder die von der Messfläche abgestrahlte Wärmestrahlung mit einem flächig ablenkbaren Scanner auf ein optische Wärmemesselement leiten, welches im Verhältnis zum gesamten Messflächendurchmesser einen kleinen Öffnungswinkel hat Mit dieser Messmethode lassen sich dann beispielsweise krankhafte Gewebebereiche ermitteln, da deren Durchblutung und damit Erwärmung bzw. Wärmekapazität sich von umliegenden Bereichen signifikant unterscheiden.

Man kann nun Messfläche und Beeinflussungsfläche derart einstellen, dass, wie oben beschrieben, sie zentrisch zueinander liegen, d.h. sich die optische Achse des Abbildungssystems **5** mit der Achse des Blasstrahls in der Mitte der Messfläche auf der auszumessenden Oberfläche schneiden. Man kann aber auch eine seitliche Verschiebung einstellen, um somit gleichzeitig einen nicht oder nur wenig beblasenen Flächenbereich mit einem stark beblasenen zu untersuchen. Es kann die Messfläche auch derart gelegt werden, dass neben der Beeinflussungsfläche vom temperierten Gasstrahl unbeeinflusste benachbarte Gebiete temperaturmässig mit ausgemessen werden.

## Patentansprüche

1. Verfahren zur Bestimmung eines zeitlichen Temperaturänderungsvorgangs am menschlichen Auge, bei dem eine eine Messfläche **(9)** wenigstens teilweise beinhaltende Beeinflussungsfläche **(19)** auf dem Auge temperiert und der zeitliche Temperaturverlauf auf der Messfläche **(9)** berührungslos strahlenphysikalisch gemessen und aufgenommen wird, **dadurch gekennzeichnet, dass** zur Temperierung der Beeinflussungsfläche diese berührungslos mit einem vorgegeben temperierbaren Gasstrahl **(20)** beblasen wird, und der zeitliche Temperaturverlauf auf der Messfläche**(9)** in einem vorgegebenen Zeitbereich nach Erreichen eines Temperaturgleichgewichts und Ausschalten des Gasstrahls sowie auch bei dessen Einwirkung gemessen und aufgenommen wird.

2. Verfahren nach Anspruch 1, **dadurch gekennzeichnet, dass** die örtliche Temperaturverteilung in der Messfläche **(9)** ermittelt wird.

3. Verfahren nach Anspruch 1 oder 2, **dadurch gekennzeichnet, dass** bei einem Patienten eine erste berührungslose Temperaturverlaufsmessung an einem mit dem Gasstrahl **(20)** beblasenen ersten Hautbereich **(19)** als Beeinflussungsfläche und zeitgleich eine weitere Temperaturmessung an einem zweiten, vom ersten distanzierten Hautbereich vorgenommen wird, und die beiden Temperaturmessungen miteinander in einen Vergleich gebracht werden.

4. Verfahren nach einem der Ansprüche 1 bis 3, **dadurch gekennzeichnet, dass** die Temperaturmessung am menschlichen Auge vorgenommen wird, welches mit einem Fixierlicht fixiert wird.

5. Vorrichtung zur Durchführung des Verfahrens nach einem der Ansprüche 1 bis 4 mit einer strahlenphysikalischen, berührungslos messenden Temperaturmesseinheit **(1)** mit einer Detektionseinheit **(7)** zur Aufnahme der Temperatur einer Messfläche **(9)** auf einer menschlichen Augenoberfläche, Mittel zur Temperierung einer Beeinflussungsfläche **(19)** auf der Augenoberfläche **(2),** wobei sich Beeinflussungs- **(19)** und Messfläche **(9)** wenigstens in einem Teilbereich überlappen und die Detektionseinheit ein optisches Abbildungssystem **(5)** hat, mit dem die Messfläche **(9)** auf der Oberfläche **(2)** auf der Detektionseinheit **(7)** abbildbar ist, **gekennzeichnet durch** einen Gasstrahl als Mittel zur Temperierung, eine Dosiereinheit **(16),** mit der der Gasstrahl **(20)** mit einem vorgegebenen Gasfluss einstell- bzw. erzeugbar ist, eine Temperiereinheit **(13),** mit der der Gasfluss temperierbar ist und eine den Gasfluss aufnehmende Blaseinheit **(3)** zur Erzeugung eines Gasstrahls **(20),** damit mit der Temperaturmesseinheit **(1)** der zeitliche Temperaturverlauf auf der temperierten Beeinflussungsfläche **(19)** bzw. einer Teilfläche der Beeinflussungsfläche **(19)** und/oder deren benachbarten Gebietsteile berührungslos strahlenphysikalisch in einem vorgegebenen Zeitbereich nach Erreichen eines Temperaturgleichgewichts und Ausschalten des Gasstrahls sowie auch bei dessen Einwirkung mess- und aufnehmbar ist.

6. Vorrichtung nach Anspruch 5, **dadurch gekennzeichnet, dass** die Temperaturmesseinheit **(1)** derart ausgebildet ist, dass mit ihr eine örtliche Temperaturverteilung in der Messfläche **(9)** ermittelbar ist.

7. Vorrichtung nach Anspruch 5 oder 6, **gekennzeichnet durch** eine mechanische Verbindungseinheit **(30)** zur gemeinsamen Fixierung von Blaseinheit **(3)** und Temperaturmesseinheit **(1)** sowie einem mit der Verbindungseinheit **(30)** mechanisch verbundenen Abstandshalter **(29)** mit einem Auflageelement **(31),** der derart ausgebildet ist, dass bei einer Auflage des Auflageelements **(31)** auf der Oberfläche **(2)** oder einem zur Oberfläche **(2)** referenzierten Referenzort sich wenigstens eine teilweise Überlappung zwischen Mess- **(9)** und Beeinflussungsfläche **(19)** ergibt, und das Abbildungssystem **(5)** derart ausgebildet und angeordnet ist, dass die Messfläche **(9)** auf der Oberfläche **(2)** mit vorgegebenen Abmessungen auf der Detektionseinheit **(7)** abgebildet wird.

8. Vorrichtung nach Anspruch 6 oder 7, **gekennzeichnet durch** eine zweite Temperaturmesseinheit und eine Auswerteeinheit, mit der die Messwerte der ersten Temperaturmesseinheit **(1)** mit denjenigen der zweiten verglichen werden.

9. Vorrichtung nach Anspruch 7 oder 8, **gekennzeichnet durch** eine Kopfstütze und ein Fixierlicht zur Fixierung des menschlichen Auges und eine mit der Kopfstütze mechanisch in Verbindung stehende Halterung zur Befestigung der Verbindungseinheit **(30)**.

10. Vorrichtung nach einem der Ansprüche 5 bis 9, **gekennzeichnet durch** einen optischen Zielstrahl zur Kennzeichnung der Messfläche **(9)**, bevorzugt zur Kennzeichnung der Flächenmitte, insbesondere zur Kennzeichnung der gesamten Fläche sowie in vorteilhafter Weise eine auf der Messfläche erkennbare optische Kennzeichnung, welche eine scharfe Abbildung der detektierenden Fläche der Detektionseinheit **(7)** auf der Messfläche **(9)** angibt.

## Claims

1. Method for determining a temporal temperature variation process at the human eye, in which method an influence region (19) on the eye, which region at least partially contains a measurement region (9), is brought to a specific temperature and the temporal temperature characteristic of the measurement region (9) is measured in a contactless radiometric manner and recorded, **characterised in that**, in order to bring the influence region to a specific temperature, a gas jet (20) which can be brought to a predetermined specific temperature is blown onto the influence region in a contactless manner, and the temporal temperature characteristic of the measurement region (9) is measured, and recorded, in a predetermined time interval after a temperature equilibrium has been reached and after the gas jet has been switched off, and also during the action of the gas jet.

2. Method according to claim 1, **characterised in that** the local temperature distribution in the measurement region (9) is ascertained.

3. Method according to claim 1 or 2, **characterised in that**, in a patient, a first contactless temperature characteristic measurement is carried out on a first skin region (19) onto which the gas jet (20) has been blown and which acts as the influence region, and, at the same time, a further temperature measurement is carried out on a second skin region spaced from the first, and the two temperature measurements are compared with one another.

4. Method according to any one of claims 1 to 3, **characterised in that** the temperature measurement is carried out on the human eye, the position of which is fixed by a fixing light.

5. Apparatus for carrying out the method according to any one of claims 1 to 4, having a radiometric temperature measuring unit (1) which measures in a contactless manner and which has a detection unit (7) for recording the temperature of a measurement region (9) on a human eye surface, means for bringing to a specific temperature an influence region (19) on the eye surface (2), the influence region (19) and the measurement region (9) overlapping one another at least in a partial area and the detection unit having an optical imaging system (5) by means of which the measurement region (9) on the surface (2) can be imaged onto the detection unit (7), **characterised by** a gas jet as means for bringing to a specific temperature, a metering unit (16) by means of which the gas jet (20) can be adjusted or generated with a predetermined gas flow, a temperature-control unit (13) by means of which the gas flow can be brought to a specific temperature, and a blowing unit (3) accommodating the gas flow and generating a gas jet (20) so that, by means of the temperature measuring unit (1), the temporal temperature characteristic of the influence region (19), which has been brought to a specific temperature, or of a partial region of the influence region (19) and/or of the adjacent areas thereof, can be measured in a contactless radiometric manner, and recorded, in a predetermined time interval after a temperature equilibrium has been reached and after the gas jet has been switched off, and also during the action of the gas jet.

6. Apparatus according to claim 5, **characterised in that** the temperature measuring unit (1) is constructed in such a manner that it can be used to ascertain a local temperature distribution in the measurement region (9).

7. Apparatus according to claim 5 or 6, **characterised by** a mechanical connecting unit (30) for the common fixing in position of the blowing unit (3) and the temperature measuring unit (1) and also a spacer (29) connected mechanically to the connecting unit (30), which spacer (29) has a support member (31) and is in a form such that, when the support member (31) rests on the surface (2) or on a reference site associated with the surface (2), at least a partial overlapping of the measurement region (9) and the influence region (19) results, and the imaging system (5) is so constructed and arranged that the measurement region (9) on the surface (2) is imaged with predetermined dimensions onto the detection unit (7).

8. Apparatus according to claim 6 or 7, **characterised by** a second temperature measuring unit and an evaluation unit by means of which the measured values of the first temperature measuring unit (1) are compared with those of the second.

9. Apparatus according to claim 7 or 8, **characterised by** a head support and a fixing light for fixing the position of the human eye, and a holder for securing the connecting unit (30), which holder is connected mechanically to the head support.

10. Apparatus according to any one of claims 5 to 9, **characterised by** an optical collimator ray for identifying the measurement region (9), preferably for identifying the centre of the region, especially for identifying the entire region, and advantageously an optical identification which is recognisable on the measurement region and which indicates a sharp image of the detecting surface of the detection unit (7) on the measurement region (9).

## Revendications

1. Procédé pour déterminer un processus de variation dans le temps de température sur l'oeil humain, dans lequel une surface d'influence (19) contenant au moins partiellement une surface de mesure (9) sur l'oeil est mise à température et la courbe de température dans le temps est mesurée et enregistrée sur la surface de mesure (9) sans contact par physique des rayonnements, **caractérisé en ce que**, pour la mise en température de la surface d'influence, on souffle sur celle-ci sans contact avec un jet de gaz (20) pouvant être mis à une température prédéfinie, et la courbe de température dans le temps sur la surface de mesure (9) est mesurée et enregistrée dans une plage de temps prédéfinie après avoir atteint un équilibre de température et avoir arrêté le jet de gaz et également au cours de son effet.

2. Procédé selon la revendication 1, **caractérisé en ce que** la répartition locale de la température dans la surface de mesure (9) est déterminée.

3. Procédé selon la revendication 1 ou 2, **caractérisé en ce qu'**on effectue une première mesure sans contact de la courbe de température chez un patient sur une première zone de peau principale (19), sur laquelle on a soufflé avec le jet de gaz (20), comme surface d'influence et en même temps une autre mesure de température sur une seconde zone de peau principale espacée de la première, et les deux mesures de température sont comparées l'une avec l'autre.

4. Procédé selon l'une quelconque des revendications 1 à 3, **caractérisé en ce que** la mesure de température est effectuée sur l'oeil humain, qui est fixé avec une lampe de fixation.

5. Dispositif pour l'application du procédé selon l'une quelconque des revendications 1 à 4 avec une unité de mesure de température (1) mesurant sans contact et par la physique des rayonnements équipée d'une unité de détection (7) pour l'enregistrement de la température d'une surface de mesure (9) sur une surface d'oeil humain, de moyens pour la mise en température d'une surface d'influence (19) sur la surface de l'oeil (2), la surface d'influence (19) et la surface de mesure (9) se chevauchant au moins dans une zone partielle et l'unité de détection ayant un système de représentation optique (5), avec lequel la surface de mesure (9) sur la surface (2) peut être représentée sur l'unité de détection (7), **caractérisé par** un jet de gaz comme moyen pour la mise en température, une unité de dosage (16), avec laquelle le jet de gaz (20) peut être réglé et généré avec un flux de gaz prédéfini, une unité de mise en température (13), avec laquelle le flux de gaz peut être mis en température et une unité de soufflage (3) recevant le flux de gaz pour générer un jet de gaz (20), afin que, avec l'unité de mesure de température (1), la courbe de température dans le temps sur la surface d'influence (19) mise en température ou sur une surface partielle de la surface d'influence (19) et/ou sur des parties de zones voisines de cette surface puisse être mesurée et enregistrée sans contact et par la physique des rayonnements dans une plage de temps prédéfinie après avoir atteint un équilibre de température et arrêté le jet de gaz et également lors de son effet.

6. Dispositif selon la revendication 5, **caractérisé en ce que** l'unité de mesure de température (1) est conçue de telle sorte qu'elle permet de déterminer une répartition locale de température dans la surface de mesure (9).

7. Dispositif selon la revendication 5 ou 6, **caractérisé par** une unité de liaison (30) mécanique pour la fixation commune de l'unité de soufflage (3) et de l'unité de mesure de température (1) et d'un écarteur (29) relié de façon mécanique à l'unité de liaison (30) avec un élément de positionnement (31), qui est conçu de telle sorte que, avec un emplacement de l'élément de positionnement (31) sur la surface (2) ou sur un lieu de référence référencé par rapport à la surface (2), on obtient au moins un chevauchement partiel entre la surface de mesure (9) et la surface d'influence (19), et le système de représentation (5) est conçu et disposé de telle sorte que la surface de mesure (9) sur la surface (2) est représentée avec des dimensions prédéfinies sur l'unité de détection (7).

8. Dispositif selon la revendication 6 ou 7, **caractérisé par** une seconde unité de mesure de température et une unité d'analyse, avec laquelle les valeurs de mesure de la première unité de mesure de température (1) sont comparées avec celles de la seconde.

9. Dispositif selon la revendication 7 ou 8, **caractérisé par** un appui-tête et une lumière de fixation pour la fixation de l'oeil humain et un support, en liaison mécanique avec l'appui-tête, pour la fixation de l'unité de liaison (30).

10. Dispositif selon l'une quelconque des revendications 5 à 9, **caractérisé par** un faisceau cible optique pour le marquage de la surface de mesure (9), de préférence pour le marquage du centre de la surface, en particulier pour le marquage de l'ensemble de la surface et de manière avantageuse un marquage visuel perceptible sur la surface de mesure, qui donne une représentation nette de la surface détectrice de l'unité de détection (7) sur la surface de mesure (9).
